# EUROPEAN PATENT SPECIFICATION

(11) **EP 4 400 079 B1**
(45) Date of publication and mention of the grant of the patent: **07.10.2026**
(21) Application number: 24151636.8
(22) Date of filing: 12.01.2024
(51) Int. Cl.: A61F 2/30, A61B 34/10, A61F 2/38, A61F 2/46

(54) **CREATION OF A HEALTHIER PATELLOFEMORAL JOINT**
ERZEUGUNG EINES GESUNDHEITSERHALTENDEN PATELLOFEMORALEN GELENKS
CRÉATION D'UNE ARTICULATION PATIOMORALE SANTE

(30) Priority: 13.01.2023 SE 2350027; 13.01.2023 SE 2351216
(43) Date of publication of application: 17.07.2024
(73) Proprietor: Episurf IP-Management AB, 114 49 Stockholm (SE)
(72) Inventor: BRATT, Ingrid, 187 34 Täby (SE); JULIN, Johan, 125 70 Älvsjö (SE); RYD, Leif, 211 42 Malmö (SE)
(74) Representative: Rouse AB

(56) References cited:
- WO-A1-2022/076773
- WO-A1-2024/091549
- US-A1- 2012 209 394
- US-A1- 2022 133 484

## Description

### TECHNICAL FIELD

The present disclosure relates generally to the turning of a patient's dysplastic or deformed patellofemoral joint into a healthier patellofemoral joint.

### BACKGROUND

Pain and overuse disorders of the joints of the body is a common problem, and one of the most important joints which is liable to wear and disease is the knee. The knee provides support and mobility, and is the largest and strongest joint in the body. The knee joint consists of four bones (femur, tibia, patella and fibula) with three articulations: the tibiofemoral articulation (between the convex femoral condyles and the concave tibial condyles), the patellofemoral articulation (where the patella lies in the intercondylar groove of the femoral trochlea), and the tibiofibular articulation (between the tibia and the fibula). The tibia, femur and patella are covered in articular cartilage, and the normal function of the knee joint depends upon this. In a healthy joint, the articulating surfaces of the interacting bone structures are shaped so as to create a stable joint, where friction between the cartilage and the surrounding parts of the joint is very low, which facilitates movement of the joints under high pressure.

The advantages of using implants for repairing damaged cartilage and/or bone in joints have stimulated the development of small joint implants, suitable for repair of injuries to cartilage and/or underlying bone that have a minimal influence on the surrounding parts of the joint. Such small implants are often designed with a mushroom-like shape, with an implant body that may be formed as a plate with a wear resistant articulating surface for facing the articulate side of the joint, and a bone contacting surface for facing the bone below the damaged part of cartilage, with a peg, rod, or screw projecting from the bone contacting side of the implant body for fastening the implant to the bone.

WO 2022/076773 A1 discloses methods, systems and apparatuses for assessing a patella in a patient-specific manner and automating various calculations in order to optimise patellar implant selection and placement for a particular patient, thereby reducing the likelihood of postoperative pain or complications.

### PROBLEMS WITH THE PRIOR ART

When an implant is used for repairing damaged cartilage and/or bone in a patellofemoral joint, the articulating surface of the implant is typically designed to mimic the curvature of the original, undamaged, articulating surface of the patella and/or the corresponding articular surface of the femoral trochlea. However, in situations where the original curvature of the articular surface of the patella, and/or the corresponding articular surface of the femoral trochlea, is dysplastic or deformed, an implant which is designed to mimic the curvature of the original, undamaged, articulating surface will not create a healthy patellofemoral joint.

Therefore, there is a need for a method for turning of a patient's dysplastic or deformed patellofemoral joint into a healthier patellofemoral joint.

### SUMMARY

The above described problem is addressed a system for turning an patient's dysplastic or deformed patellofemoral joint into a healthier patellofemoral joint according to the invention as defined in claim 1.

The above described problem is also addressed by a method for creating an implant for turning a patient's dysplastic or deformed patellofemoral joint into a healthier patellofemoral joint according to the invention as defined in claim 7.

This enables the use of implants for improving dysplastic or deformed patellofemoral joints.

In embodiments, the determination of whether any of the analyzed curvatures indicate that the patellofemoral joint is dysplastic or deformed involves determining whether the sulcus angle between the medial and lateral trochlear facets of the femur is larger than a threshold, which may e.g. be 145-160 degrees, preferably 145-150 degrees. This is a structured way of determining whether any of the analyzed curvatures indicate that the patellofemoral joint is dysplastic or deformed. However, an experienced radiologist may be able to determine whether the patellofemoral joint is dysplastic or deformed without having to actually measure the sulcus angle, although such a measurement may form a part of this determination. A radiologist may e.g. when analyzing curvatures in a patellofemoral joint determine that an angle indicates a dysplastic or deformed patellofemoral joint, even without actually measuring any angles. **An** actual measurement of the sulcus angle typically involves placing the angle tip at the lowest point of the intercondylar groove of the femoral trochlea.

In embodiments, the sulcus angle is measured in radiological images (e.g. images provided by a magnetic resonance imaging (MRI) system, an x-ray imaging system, an ultrasonic imaging system, a fluoroscopic imaging system and/or a computer tomography (CT), e.g. a CBCT or Arthro-CT, system), or in a 3D model generated based on a series of radiological images. However, the sulcus angle may also be measured on the physical bone. The above described system and method may thus be used for turning a patient's dysplastic or deformed patellofemoral joint into a healthier patellofemoral joint by performing the steps in advance of surgery, or by performing the steps during surgery, when the bone may be made accessible for physical measurement of the sulcus angle before the implant shape and dimensions are determined. This may be especially advantageous for robot surgery, especially if the implant is during surgery selected from a predefined set of standardized implants having varying dimensions.

In embodiments, if there is determined to be damage to the patella, the curvature of the articulating surface of the patella that is analyzed is a simulated surface corresponding to the articulating surface that the patella would have had if it had not been damaged. This enables a determination of whether the patella is dysplastic or deformed, or simply damaged. If there is determined to be damage, an analysis of the damaged curvature may not provide correct results regarding whether the patella is dysplastic or deformed, and it is therefore preferred to instead analyze a simulated healthy surface.

In embodiments, if there is determined to be damage to the femoral trochlea, the curvature of the articulating surface of the femoral trochlea that is analyzed is a simulated surface corresponding to the articulating surface that the femoral trochlea would have had if it had not been damaged. This enables a determination of whether the femoral trochlea is dysplastic or deformed, or simply damaged. If there is determined to be damage, an analysis of the damaged curvature may not provide correct results regarding whether the femoral trochlea is dysplastic or deformed, and it is therefore preferred to instead analyze a simulated healthy surface.

In embodiments, example curvatures of articulating surfaces in a healthy patellofemoral joint are stored in a storage means. Such example curvatures of articulating surfaces in a healthy patellofemoral joint may be retrieved from the storage means, and used to propose an improved curvature of the articulating surface of the patella and/or the femoral trochlea based on the retrieved curvatures.

The above described problem is also addressed by a non-transitory machine-readable medium on which is stored machine-readable code which, when executed by a processor, controls the processor to perform any one of the above described methods.

The above described problem is further addressed by a system for determining the shape and dimensions of at least one implant for turning a patient's dysplastic or deformed patellofemoral joint into a healthier patellofemoral joint. The system preferably comprises at least one processor arranged to: generate a first 3D model based on a series of radiological images of the patellofemoral joint; adapt the first 3D model into a second 3D model, by adapting a surface curvature of the first 3D model into a desired surface curvature; determine the shape and dimensions for at least one implant based on the second 3D model; and ensure that all the points around the circumference of said implant in the second 3D model correspond to points on the surface of the first 3D model. In embodiments, the at least one processor is further arranged to determine the shape and dimensions for at least one guide tool based on the first 3D model.

The above described problem is also addressed by a method for determining the shape and dimensions of at least one implant for turning a patient's dysplastic or deformed patellofemoral joint into a healthier patellofemoral joint. The method preferably comprises: generating a first 3D model based on a series of radiological images of the patellofemoral joint; adapting the first 3D model into a second 3D model, by adapting a surface curvature of the first 3D model into a desired surface curvature; determining the shape and dimensions for at least one implant based on the second 3D model; and ensuring that all the points around the circumference of said implant in the second 3D model correspond to points on the surface of the first 3D model. In embodiments, the method further comprises determining the shape and dimensions for at least one guide tool based on the first 3D model.

In embodiments, account is also taken to determined damage to the patellofemoral joint when determining the shape and dimensions for a patellar implant and/or a trochlear implant, e.g. by adapting parameters such as the implant area, the implant thickness, and/or the positioning of the implant.

In embodiments, the determined shape and dimensions of the implant or implants are output as parameters for manufacturing said implant or implants. This enables the manufacturing of implants suitable for turning a patient's dysplastic or deformed patellofemoral joint into a healthier patellofemoral joint.

The processor may in some embodiments comprise several different processors which together perform the claimed functions. The fact that a certain step is effected by at least one processor does not mean that the step is necessarily entirely automatic - the at least one processor may effect a step using manual input.

The scope of the invention is defined by the claims. A more complete understanding of embodiments of the invention will be afforded to those skilled in the art, as well as a realization of additional advantages thereof, by a consideration of the following detailed description of one or more embodiments. Reference will be made to the appended sheets of drawings that will first be described briefly.

### BRIEF DESCRIPTION OF THE DRAWINGS

Fig. 1 shows a schematic view of a system for turning a patient's dysplastic or deformed patellofemoral joint into a healthier patellofemoral joint, in accordance with one or more embodiments described herein.
Figs. 2a-b schematically illustrate the sulcus angle in a healthy and in a dysplastic or deformed femoral trochlea, respectively, in accordance with one or more embodiments described herein.
Figs. 3a-b schematically illustrate how a dysplastic or deformed patellofemoral joint may be made healthier through the use of a trochlear implant, in accordance with one or more embodiments described herein.
Figs. 4a-d illustrate a patellar implant, in accordance with one or more embodiments described herein.
Figs. 5a-c illustrate embodiments of a trochlear implant and a trochlear guide tool, in accordance with one or more embodiments described herein.
Figs. 6a-c illustrate a patellar guide tool, Figs. 6d illustrates the drilling of a recess for a patellar implant, and Fig. 6e illustrates a surgical kit, in accordance with one or more embodiments described herein.
Figs. 7a-c illustrate a patellofemoral implant arrangement for turning a patient's dysplastic or deformed patellofemoral joint into a healthier patellofemoral joint, in accordance with one or more embodiments described herein.
Fig. 8 is a schematic flow diagram for a method for turning a patient's dysplastic or deformed patellofemoral joint into a healthier patellofemoral joint, in accordance with one or more embodiments described herein.
Fig. 9 is a schematic flow diagram for a method for determining the shape and dimensions of at least one implant for turning a patient's dysplastic or deformed patellofemoral joint into a healthier patellofemoral joint, in accordance with one or more embodiments described herein.

Embodiments of the present disclosure and their advantages are best understood by referring to the detailed description that follows. It should be appreciated that like reference numerals are used to identify like elements illustrated in one or more of the figures.

### DETAILED DESCRIPTION

### Introduction

The primary function of the patella is to work as a mechanical pulley increasing the efficacy of the quadriceps muscle. The patella is a part of a gliding joint and performs movements in several directions. During a normal knee flexion, the patella first glides medially to center itself in the trochlear groove while tilting 5-7 degrees laterally. Further in the flexion, the patella tracks over to the lateral side. A normal patella glides about 3 mm medially and laterally from its center point during motion.

The stress on the articulating tissue is dependent on the contact area between patella and femur. Small contact area and high applied forces result in high patellofemoral stresses which can be harmful for the cartilage. During knee flexion, different articulating patella surface areas are in contact with the femoral cartilage. In situations where the original curvature of the articular surface of the patella, and/or the corresponding articular surface of the femoral trochlea, is dysplastic or deformed, the contact area is often reduced, and the joint is also typically less stable than it should be. This may cause patellar dislocation and/or patellar fracture.

The present disclosure relates generally to a system and a method for turning of a patient's dysplastic or deformed patellofemoral joint into a healthier patellofemoral joint. Embodiments of the disclosed solution are presented in more detail in connection with the figures.

### System architecture

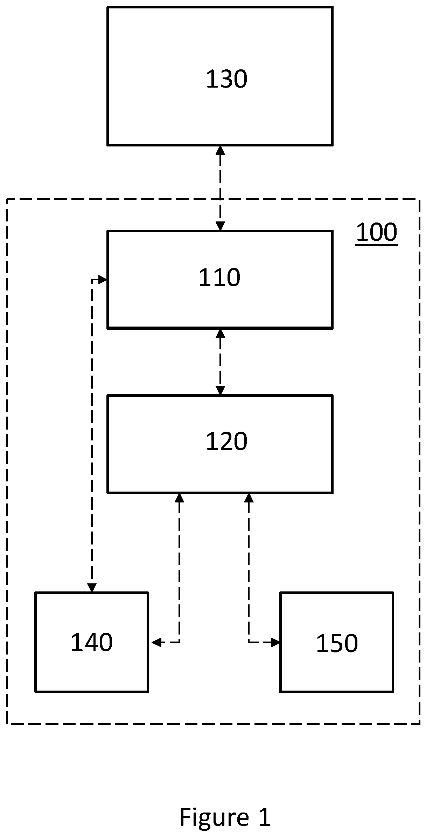

Fig. 1 shows a schematic view of a system 100 for turning a patient's dysplastic or deformed patellofemoral joint into a healthier patellofemoral joint. According to embodiments, the system 100 comprises a display 140, at least one manipulation tool 150, and a storage media 110, configured to receive and store image data and parameters. In some embodiments, the system 100 is communicatively coupled to a medical imaging system 130. The medical imaging system 130 may be configured to capture or generate medical images, e.g. radiology images such as X-ray images, ultrasound images, computed tomography (CT), e.g. CBCT or Arthro-CT, images, nuclear medicine including positron emission tomography (PET) images, and magnetic resonance imaging (MRI) images. The storage media 110 may be configured to receive and store medical images from the medical imaging system 130. In embodiments, medical images are uploaded into the storage media 110 by personnel at a medical care facility, preferably the medical care facility where the medical imaging takes place. Medical images may however also be uploaded into the storage media 110 by another medical care facility, or by other authorized personnel. The uploading of the medical images may also be an automatic uploading directly from one system to another. The display 140 may be configured to receive image data for display via the processor 120, and/or to retrieve image data for display directly from the storage media 110, possibly in response to a control signal received from the processor 120 or the at least one manipulation tool 150.

In one or more embodiments, the system 100 comprises at least one processor 120 configured to: analyze the curvature of the patella in the patellofemoral joint; analyze the curvature of the femoral trochlea in the patellofemoral joint; determine whether any of the analyzed curvatures indicate that the patellofemoral joint is dysplastic or deformed; if any of the analyzed curvatures indicate that the patellofemoral joint is dysplastic or deformed, propose an improved curvature of the articulating surface of the patella and/or the femoral trochlea; and determine the shape and dimensions for a patellar implant that would create said improved curvature of the articulating surface of the patella, and/or a trochlear implant that would create said improved curvature of the articulating surface of the femoral trochlea.

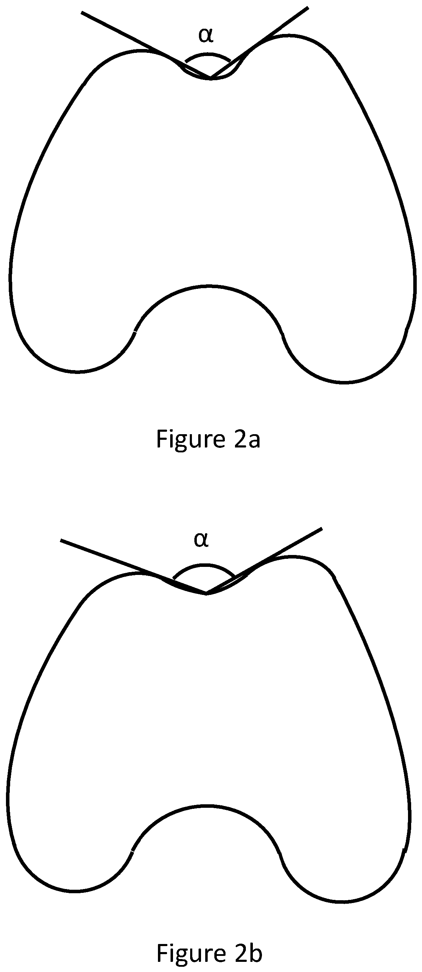

The the at least one processor 120 is configured to also output said determined shape and dimensions of the implant or implants as parameters for manufacturing said implant or implants. The determination of whether any of the analyzed curvatures indicate that the patellofemoral joint is dysplastic or deformed may e.g. involve determining whether the sulcus angle α between the medial and lateral trochlear facets of the femur is larger than a threshold. In a healthy femoral trochlea, as schematically illustrated in Fig. 2a, the sulcus angle α is typically 138±6 degrees. This means that the intercondylar groove of the femoral trochlea, in which the patella lies, is relatively deep. This makes the patellofemoral joint stable, and ensures that the patella is secured in position. If the sulcus angle α is much larger, as schematically illustrated in Fig. 2b, this means that the intercondylar groove of the femoral trochlea, in which the patella lies, is shallow. This makes the patellofemoral joint much less stable, and increases the risk of patellar subluxation, where the patella becomes dislocated. In extreme cases, the sulcus angle may even be >180 degrees, so that instead of an intercondylar groove, there is an intercondylar protrusion. The patellofemoral joint may therefore be considered to be dysplastic or deformed if the sulcus angle α is larger than for a healthy patellofemoral joint.

In order to determine this, the sulcus angle α may be compared with a suitable threshold. If the threshold is set to 145-160 degrees, preferably 145-150 degrees, a comparison of the sulcus angle α with this threshold will provide a good indication of whether the patellofemoral joint is dysplastic or deformed. Correspondingly, the determination of whether any of the analyzed curvatures indicate that the patellofemoral joint is dysplastic or deformed may involve a comparison of a suitable patellar angle with a suitable threshold, which may e.g. be whether the patellar angle is smaller than 119 degrees (the patellar angle is typically 129,5±10 degrees). However, a radiologist may be able to determine whether the patellofemoral joint is dysplastic or deformed based on experience, without having to actually measure any angles (although such a measurement may form a part of this determination). A radiologist may e.g. when analyzing curvatures in a patellofemoral joint determine that an angle indicates a dysplastic or deformed patellofemoral joint, even without actually measuring any angles. An actual measurement of the sulcus angle typically involves placing the angle tip at the lowest point of the intercondylar groove of the femoral trochlea.

When the patellofemoral joint is dysplastic or deformed, there is also an increased risk of damage to the patellofemoral joint, such as e.g. cartilage lesions. It is therefore common that the realization that the patellofemoral joint is dysplastic or deformed only comes when there is also damage to the patellofemoral joint. In this case, the same implant or implants may be used for damage repair as well as for improving the surface curvature. The at least one processor 120 may therefore be arranged to take into account also of determined damage to the patellofemoral joint when determining the shape and dimensions for the implant or implants. It may be desirable to ensure that any determined damage to the patellofemoral joint will be repaired by the implant, by adapting parameters such as the implant area, the implant thickness, and/or the positioning of the implant.

In situations where there is damage to the cartilage in the patella, it may be advantageous not to analyze the actual, damaged, articulating surface, but instead first simulate the "original" articulating surface, that the patella would have had if it had not been damaged. If there is determined to be damage, an analysis of the damaged curvature may not provide correct results regarding whether the patella is dysplastic or deformed, and it is therefore preferred to instead analyze a simulated healthy surface. If this surface is analyzed, this gives a better indication of whether the patellofemoral joint is dysplastic or deformed. This enables a determination of whether the patella is dysplastic or deformed, or just simply damaged.

In the same way, if there is damage to the cartilage in the femoral trochlea, it may be advantageous not to analyze the actual, damaged, articulating surface, but instead first simulate the "original" articulating surface that the femoral trochlea would have had if it had not been damaged. If there is determined to be damage, an analysis of the damaged curvature may not provide correct results regarding whether the femoral trochlea is dysplastic or deformed, and it is therefore preferred to instead analyze a simulated healthy surface. If this surface is analyzed, this gives a better indication of whether the patellofemoral joint is dysplastic or deformed. This enables a determination of whether the femoral trochlea is dysplastic or deformed, or just simply damaged.

The "original" articulating surface may e.g. be simulated based on the determined surface curvature of the cartilage and/or the subchondral bone in a predetermined area comprising and surrounding the determined damage. **It** is desirable to simulate the "original" articulating surface as closely as possible. If just the 3D curvature of the subchondral bone subjacent to the area of damaged cartilage is used for simulating the "original" articulating surface, this does not necessarily mimic the original, undamaged, articulating surface, since the cartilage does not necessarily have uniform thickness. However, for anatomical parts where the cartilage does have a substantially uniform thickness, the 3D curvature of the subchondral bone subjacent to the area of damaged cartilage may be used for simulating the "original" articulating surface.

According to embodiments, the "original" articulating surface is instead simulated based on the curvature of the cartilage surrounding the area of damaged cartilage. Preferably, a suitable area comprising and extending around the damaged cartilage is selected, and the curvature of the whole area is simulated in such a way that the curvature of the area which is not damaged matches the actual curvature. The simulation may comprise an interpolation, e.g. using the Solid Works Surface Wizard or another suitable tool.

It may be advantageous to double-check that the simulated articulating surface of the patella matches the simulated articulating surface of the opposing femoral trochlea, since the articulating surfaces of the patella and the femoral trochlea would normally match each other in a patellofemoral joint. However, in a dysplastic or deformed patellofemoral joint, this is not always the case.

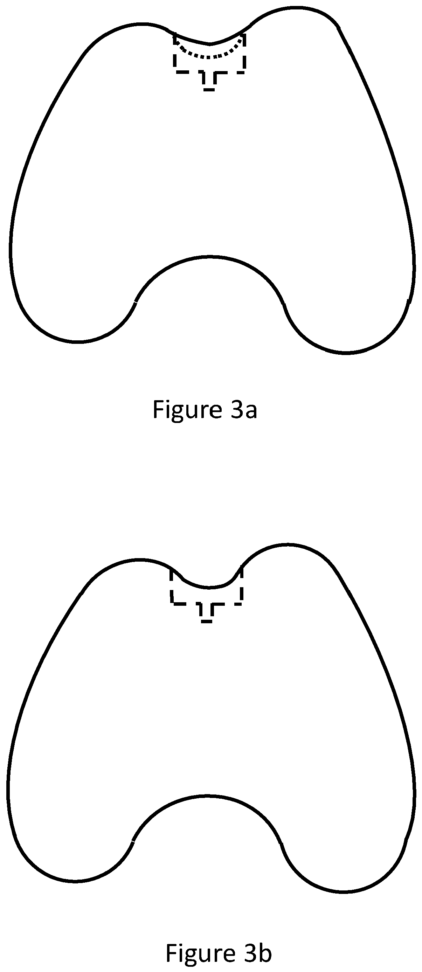

When it has been determined that the patellofemoral joint is dysplastic or deformed, it may be possible to design one or more implants that will make the patellofemoral joint healthier by changing the curvature of at least one of the articulating surfaces. Fig. 3a-b schematically illustrate how this could be done.

In Fig. 3a, the dashed line schematically illustrates the shape of a recess that could be e.g. drilled or milled in a dysplastic or deformed femoral trochlea in order to change the curvature of the articulating surface. The dotted line illustrates the desired surface curvature of the femoral trochlea. Fig. 3b schematically illustrates how the curvature of the articulating surface may be changed by placing an implant having the desired surface curvature in the recess of Fig. 3a. In this way, a dysplastic or deformed patellofemoral joint may be made healthier through the use of a trochlear implant having the desired surface curvature.

If the articulating surface of the patella is dysplastic or deformed, it is possible to in the same way change the curvature of the articulating surface of the patella through the use of a patellar implant having the desired surface curvature. It is also possible to use a patellofemoral implant arrangement, comprising a femoral trochlear implant as well as a patellar implant. The implants would then preferably comprise articulating surfaces that are designed to allow that they at least partly interact with each other when the implants are implanted into the knee joint. The curvatures of the articulating surface of the patellar implant is then preferably designed to match the articulating surface of the opposing femoral trochlear implant, since this makes the patellofemoral joint healthier.

In order to design a suitable implant for the patella (or the femoral trochlea) with a suitable guide tool, it may be desirable to use two different 3D models, a first 3D model that corresponds to the actual surface curvature of the cartilage (actual model) and/or the subchondral bone, and a second 3D model that corresponds to the desired surface curvature of the cartilage and/or the subchondral bone (desired model). The "actual model" would typically be generated based on a series of radiological images (e.g. images provided by a magnetic resonance imaging (MRI) system, an x-ray imaging system, an ultrasonic imaging system, a fluoroscopic imaging system and/or a computer tomography (CT), e.g. a CBCT or Arthro-CT, system) of the patellofemoral joint, as is known in the art.

The "actual model" may then be used for designing a guide tool 550, 600, since the guide tool 550, 600 should have a cartilage contact surface that has a shape and contour that is designed to correspond to and to fit the contour of the articulating surface of the patella (or the femoral trochlea) in a predetermined area comprising and surrounding the implant site, so that the guide tool 550, 600 will have a stable mounting in the correct position. This helps ensuring that an implant recess will be created in the exact desired position.

The "desired model" may be used for designing a suitable implant, that may be used to change the curvature of the articulating surface into the desired surface curvature. The "desired model" could be created by adapting the surface curvature of the "actual model" into a desired surface curvature. The desired surface curvature may e.g. be determined based on a number of "design rules" regarding parameters such as the sulcus angle, the height of the trochlear facets, whether the groove should be straight or curved, and/or the shape of the bottom of the groove (e.g. rounded or sharp). There may also be example curvatures of articulating surfaces in a healthy patellofemoral joint stored in storage means 100, so that the desired surface curvature may be determined based on such stored example curvatures.

The advantage of using two different 3D models in this way is that it can then easily be determined whether all the points around the circumference of the implant in the "desired model" correspond to points in the same positions in the "actual model". This is necessary in order for the surface to be smooth, with no sharp edges, when the implant has been implanted. A system 100 for determining the shape and dimensions of at least one implant for turning a patient's dysplastic or deformed patellofemoral joint into a healthier patellofemoral joint may thus comprise at least one processor 120 arranged to: generate a first 3D model based on a series of radiological images of the patellofemoral joint; adapt the first 3D model into a second 3D model, by adapting a surface curvature of the first 3D model into a desired surface curvature; determine the shape and dimensions for at least one implant based on the second 3D model; and ensure that all the points around the circumference of said implant in the second 3D model correspond to points on the surface of the first 3D model. In embodiments, the at least one processor 120 may further be arranged to determine the shape and dimensions for at least one guide tool based on the first 3D model.

The at least one processor 120 may for example be a general data processor, or other circuit or integrated circuit capable of executing instructions to perform various processing operations. The at least one processor 120 may in some embodiments comprise several different processors 120 which together perform the claimed functions. In the same way, the storage media 110 may in some embodiments comprise several different storage media 110 which together perform the claimed functions. The at least one processor 120 may further be configured to perform any or all of the method steps of any or all of the embodiments presented herein.

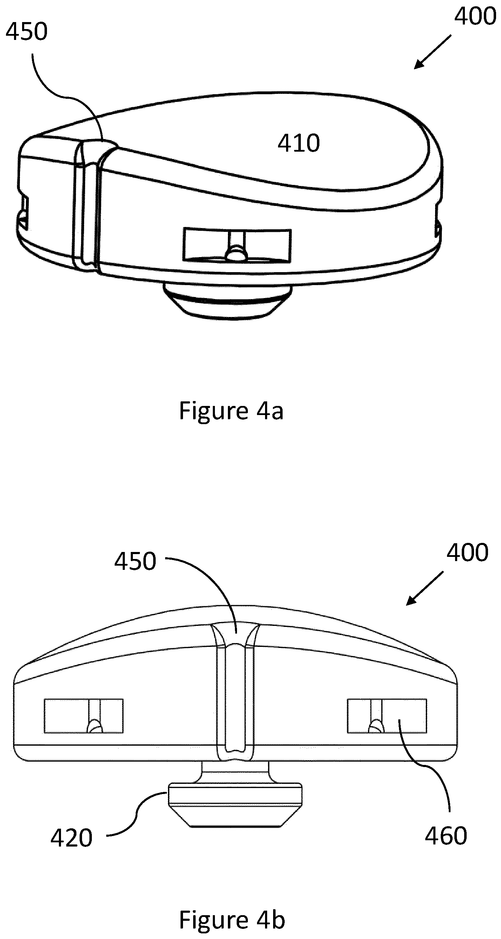

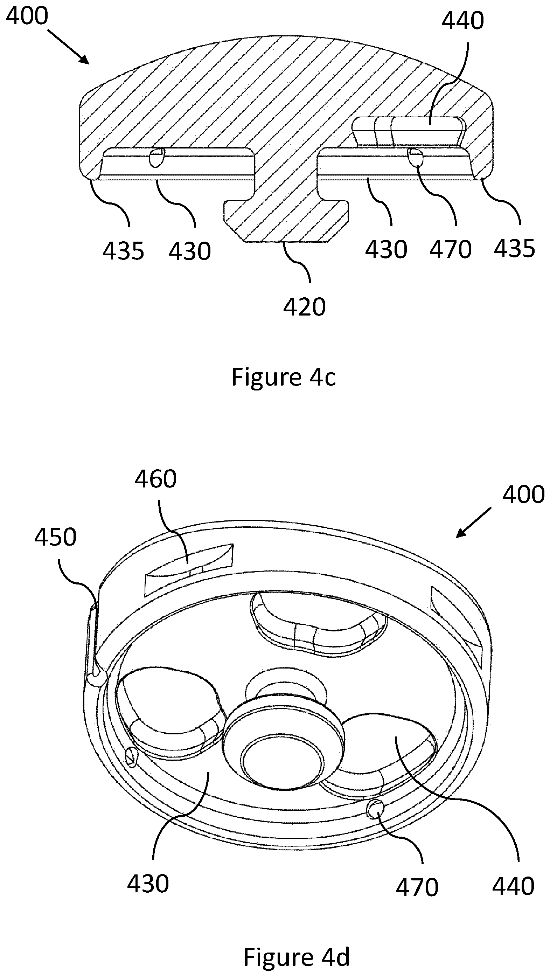

Figs. 4a-d illustrate an embodiment of a patellar in-lay implant 400 comprising an articulating surface 410. The illustrated patellar implant 400 has an approximately cylindrical shape, but the patellar implant may also have a shape that corresponds to a plurality of partly overlapping cylinders, or any other suitable shape. The patellar implant 400 may comprise an implant peg 420, extending from a suitably shaped bone contacting surface 430, which in embodiments is surrounded by an implant flange 435. The patellar implant 400 preferably comprises one or more implant cavities 440 with openings towards the bone contacting surface 430. Before the patellar implant 400 is inserted into a suitably shaped recess in the patella, the recess is preferably filled with an adhesive such as e.g. bone cement. Bone cement consists of powder, most commonly polymethylmethacrylate (PMMA), mixed with liquid. When the patellar implant 400 is inserted into the recess, the adhesive is pressed into the one or more implant cavities, and the adhesive in these implant cavities helps securing the patellar implant to the patella. If an adhesive such as bone cement is used, it may not be necessary for the patellar implant 400 to be designed for press-fit in the recess. The use of press-fit (where the implant is slightly larger than the recess) secures the patellar implant 400 to the recess regardless of whether an adhesive such as bone cement is used, but the combination of press-fit and adhesive of course secures the patellar implant 400 even more to the recess.

The patellar implant 400 may comprise one or more side recesses 460, which may have adhesive guides that guide the adhesive to the side recesses 460 when the patellar implant 400 is inserted into the recess in the patella. An adhesive guide may e.g. be in the form of a channel 470, as illustrated in Figs. 4c-d, or in the form of an indentation in the side of the patellar implant up to the side recess 460, that guides the adhesive from below the patellar implant up to the side recess 460. If the patellar implant 400 comprises an implant flange 435 surrounding the bone contacting surface 430, an adhesive guide may be a side opening 470 arranged in the implant flange 435. If there is no implant flange 435, an adhesive guide may e.g. be an opening extending from an implant cavity 440 to a side recess 460. The adhesive in the side recesses 460 helps securing the patellar implant 400 to the patella.

The patellar implant 400 may also comprise a positioning mark 450, preferably positioned on the articulating surface 410. The positioning mark 450 may in embodiments extend also to the side of the patellar implant 400.

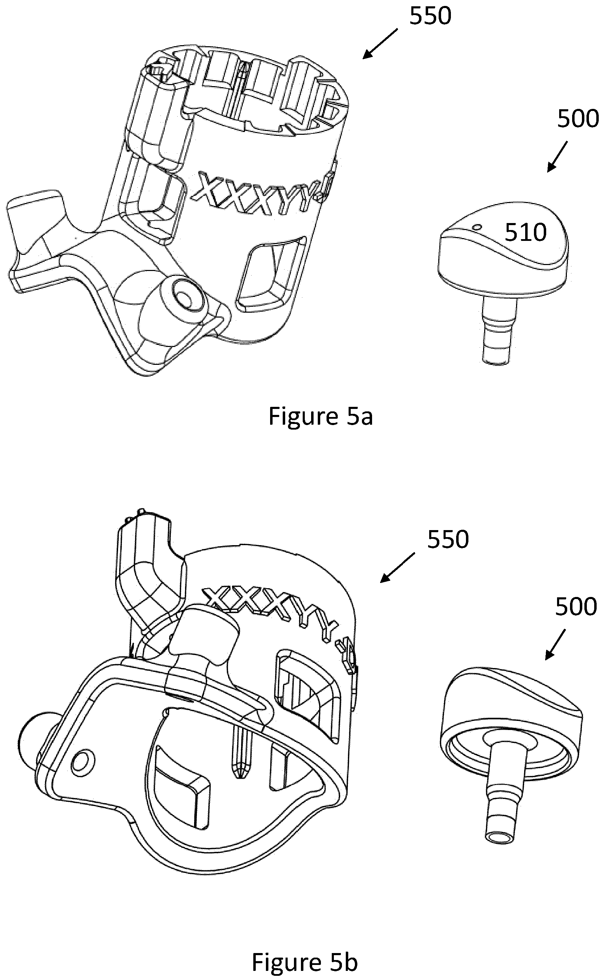

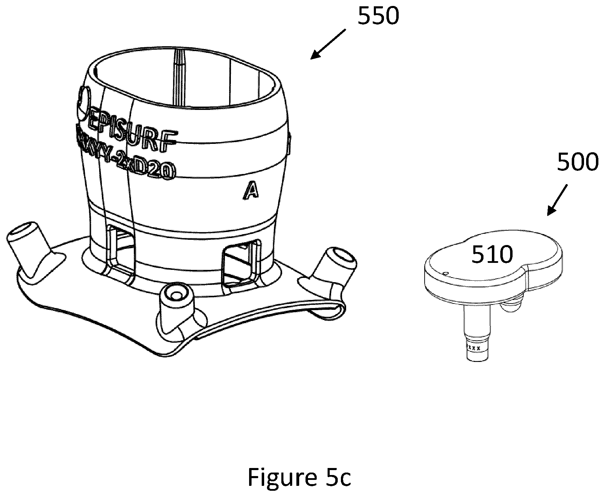

Figs. 5a-c illustrate embodiments of a trochlear implant 500 and a trochlear guide tool 550 for the femoral trochlea. Trochlear implants 500 and trochlear guide tools 550 are as such well known and described e.g. in US8655468. Fig. 5c illustrates a guide tool for a twin implant (an implant which has a shape that corresponds to two partly overlapping cylinders). Trochlear guide tools are generally quite similar to patellar guide tools. The description below of the patellar guide tool 600 thus generally applies also for the trochlear guide tool 550.

The articulating surface 510 of the femoral trochlear implant 500 is preferably a metal, metal alloy, or ceramic surface, e.g. comprising titanium (Ti) or titanium alloy, titanium nitride (TiN), titanium niobium Nitride (TiNbN), and/or a cobalt-chromium (CoCr) alloy. In order to avoid a very hard surface, such as a metal, metal alloy, or ceramic surface, interfacing with another very hard surface, creating e.g. a metal-on-metal interface, the patellar implant 400 preferably has an articulating surface 410 that is not a metal, metal alloy, or ceramic surface. The main body of the patellar implant 400 may be manufactured from metal, metal alloy, or ceramic, but the articulating surface 410 preferably comprises a polymer material, such as polyethylene, e.g. the polyethylene UHMWPE (e.g. cross-linked UHMWPE or vitamin E enhanced UHMWPE). Preferably, the whole patellar implant 400 is manufactured from the same polymer material, since this simplifies the manufacturing process.

If the bone contacting surface of the patellar implant 400 or the trochlear implant 500 is a non-porous metal, metal alloy, or ceramic surface, it may be advantageous to coat the bone contacting surface with an osseointegrating and/or bioactive material, such as e.g. hydroxyapatite. This reduces the need for using an adhesive for securing the implant 400, 500 in the recess in the bone. It may however be difficult to coat a polymer material with a bioactive material, so the use of a bioactive coating on the bone contacting surface is simplified if the body of the patellar implant 400 is manufactured from metal, metal alloy, or ceramic, even though the articulating surface 410 comprises a polymer material.

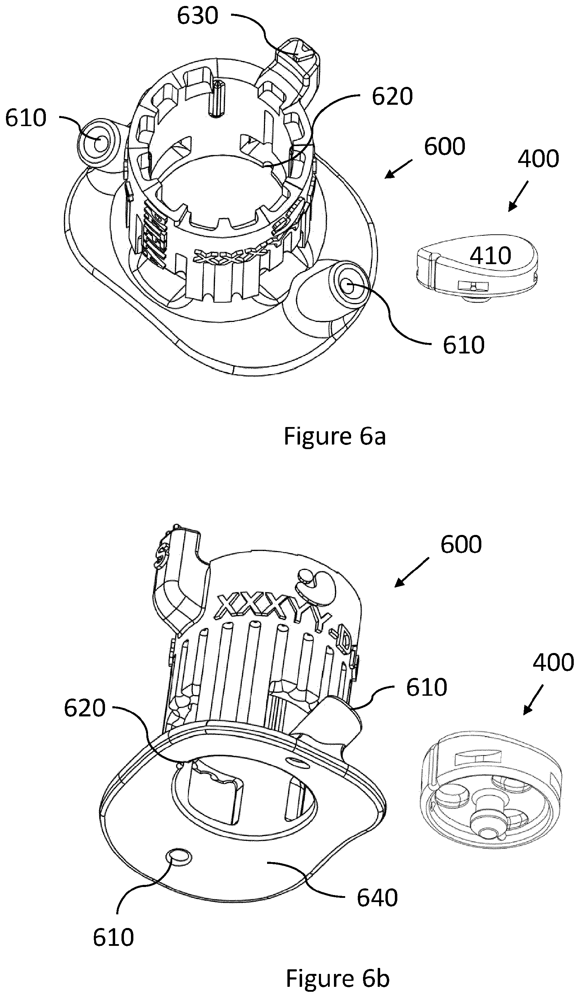

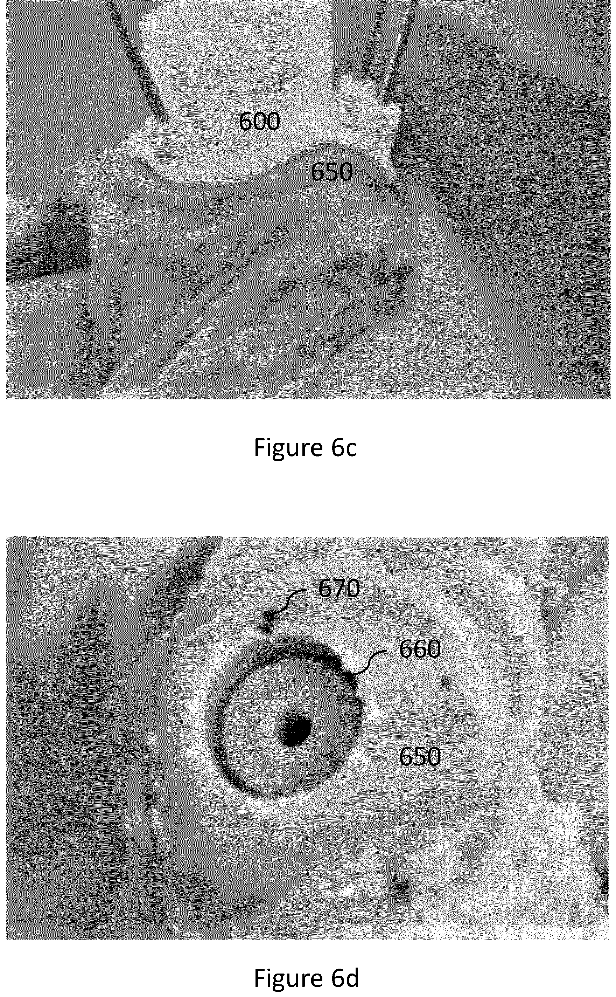

Figs. 6a-b illustrate a patellar guide tool 600, which **in** Fig. 6c is shown attached to the articulating surface of the patella 650. The guide tool 600 preferably has a cartilage contact surface 640 that has a shape and contour that is designed to correspond to and to fit the contour of the articulating surface of the patella 650 in a predetermined area comprising and surrounding the implant site. Thereby, the cartilage contact surface 640 of the guide tool 600 corresponds to and fits to the surface contour of the articulating surface of the patella 650. The whole cartilage contact surface 640 does not have to correspond to the contour curvature of the articulating surface of the patella 650, it is enough if the cartilage contact surface 640 comprises at least three cartilage contact points, so that the guide tool 600 will have a stable mounting in the correct position on the patella 650. This helps ensuring that the recess 660 will be created in the exact desired position. Such cartilage contact points are preferably chosen to provide maximum support and positional stability for the guide tool 600.

The cartilage contact surface 640 of the guide tool 600 may be further stabilized by being attached to the patella 650 with one or more nails, rivets, wires or similar attachment means, as illustrated in Fig. 6c. Such additional attachment gives additional support and stability, and enables the cartilage contact surface 640 of the guide tool 600 to be as small as possible, which is especially important for the patella 650, since it is quite small. The guide tool 600 may comprise through-holes 610, through which the one or more nails, rivets, wires or similar attachment means may be inserted, in order to attach the guide tool 600 to the patella 650.

The guide tool 600 may comprise a rotational position indicator 630, which may be used to make a marking 670 on the cartilage at the side of the recess 660 in the patella 650, as illustrated in Fig. 6d. Such a marking 670 may then be used to correctly rotate the patellar implant 400 when the patellar implant 400 is inserted into the recess 660 in the patella 650. If the patellar implant 400 comprises a positioning mark 450, the alignment of this positioning mark 450 with the marking 670 on the cartilage at the side of the recess 660 in the patella 650 ensures that the patellar implant 400 is correctly rotated in the recess 660. The guide tool 600 is preferably configured to allow such a marking 670 to be made while the guide tool 600 is attached to the patella 650. The guide tool 600 may for this purpose comprise an indentation 620 at the position of the rotational position indicator 630 on the guide tool 600. The marking 670 may e.g. be added to the cartilage surface by inserting a marking pen into the indentation 620 in the guide tool 600 when the guide tool 600 is attached to the patella 650.

A correct rotational positioning of the patellar implant 400 is important because the articulating surface 410 of the patellar implant 400 will in most situations not be rotationally symmetric. If the patellar implant 400 is not mounted with a correct rotational positioning, there may be sharp edges which may be painful for the patient.

A marking on the cartilage surface makes it easy for the surgeon to insert the patellar implant with a correct rotational positioning, if the patellar implant also comprises a positioning mark. Preferably, there is a positioning mark also on the insert tool, so that the implant engaging portion may be correctly rotated with respect to the patellar implant.

The above applies also to trochlear implants 500.

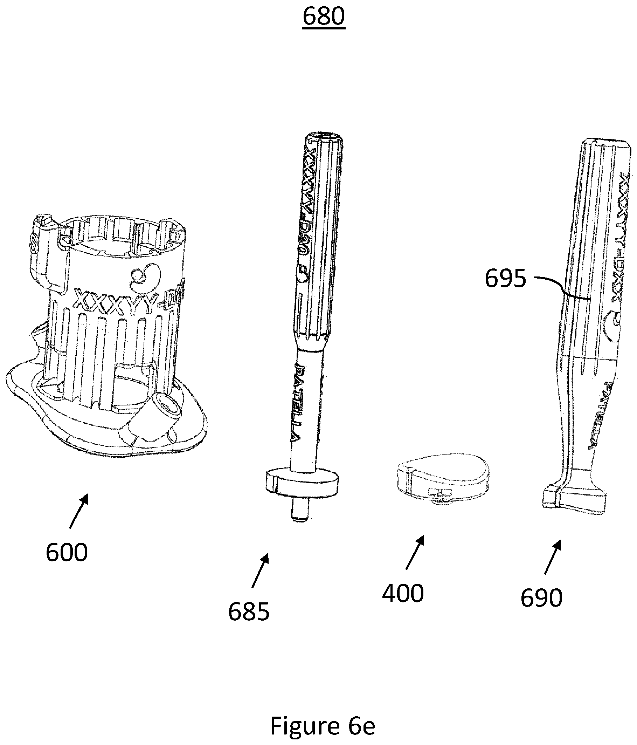

In order to create a healthier curvature in the patella 650, a patellar surgical kit 680 comprising the above described patellar implant 400, the above described patellar guide tool 600, and an insert tool may be used. Even if the patellar implant 400 would be an implant selected from a predefined set of standardized implants having varying dimensions, it is still preferred to use a customized guide tool 600, having a cartilage contact surface 640 configured to have a shape and contour that is designed to correspond to and to fit the contour of the cartilage or the subchondral bone in a predetermined area comprising and surrounding the implant site, since this will ensure that the guide tool 600 will have a stable mounting in the correct position on the patella 650. This helps ensuring that the recess 660 will be created in the exact desired position. Fig. 6e illustrates a patellar surgical kit 680 comprising a patellar guide tool 600, an implant dummy 685, a patellar implant 400, and a mandrel 690 to aid insertion of the patellar implant 400 into the recess 660.

Correspondingly, in order to create a healthier curvature in the femoral trochlea, a trochlear surgical kit comprising the above described trochlear implant 500, the above described trochlear guide tool 550, and an insert tool may be used. Even if the trochlear implant 500 would be an implant selected from a predefined set of standardized implants having varying dimensions, it is still preferred to use a customized guide tool 550, having a cartilage contact surface configured to have a shape and contour that is designed to correspond to and to fit the contour of the cartilage or the subchondral bone in a predetermined area comprising and surrounding the implant site, since this will ensure that the guide tool 550 will have a stable mounting in the correct position. This helps ensuring that the recess will be created in the exact desired position.

When there is damage and/or dysplasia in both the femur and the patella 650, a patellofemoral surgical kit may instead be used. The patellofemoral surgical kit may comprise the above described patellar implant 400, the above described trochlear implant 500, the above described patellar guide tool 600, the above described trochlear guide tool 550, and one or more insert tools. The patellar guide tool 600 for the patellar implant 400 and/or the trochlear guide tool 550 for the trochlear implant 500 preferably comprise visual markings 695, so that they are visually distinct from each other. In this way, it will be clear to the surgeon which guide tool to use for which implant.

The insert tool may e.g. be one or more mandrels 690 to aid insertion of the implant 400, 500 into the recess. A surgical kit may also comprise further instruments, such as e.g. an implant dummy 685 for verifying that the drill depth is correct before insertion of the implant, and/or one or more inserts into the patellar guide tool 600, and/or the trochlear guide tool 550, to enable precision in the drilling process. A drill bit, possibly adapted to the specific implant, may also be included. For a patellofemoral surgical kit, it is advantageous if either all the instruments associated with the trochlear implant 500 or all the instruments associated with the patellar implant 400 comprise visual markings 695, so that it is visually clear which instruments belong together.

The visual markings 695 may be any type of markings that are visible to a surgeon, such as e.g. colour markings. However, the visual markings 695 may also be tactile, such as ribs, recesses or indentations on the parts of the instruments that are held by the surgeon during use. Fig. 6e illustrates markings 695 in the form of ribs on a patellar surgical kit.

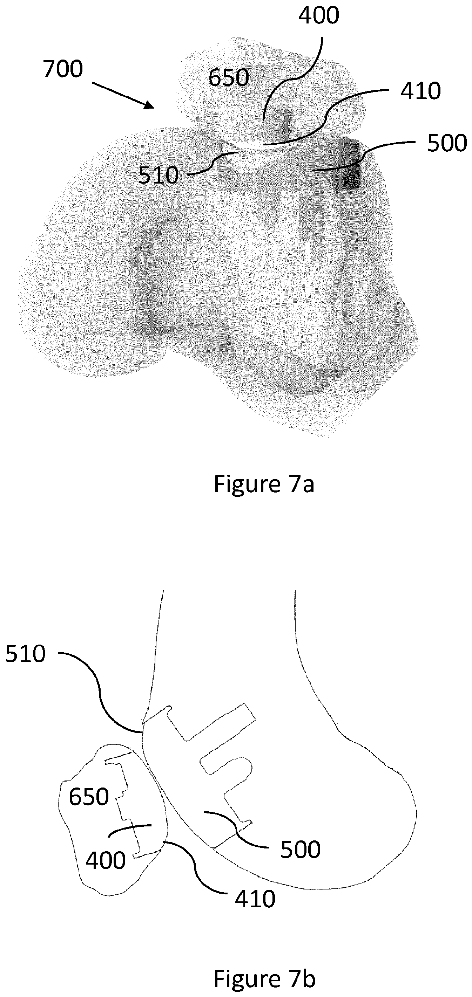

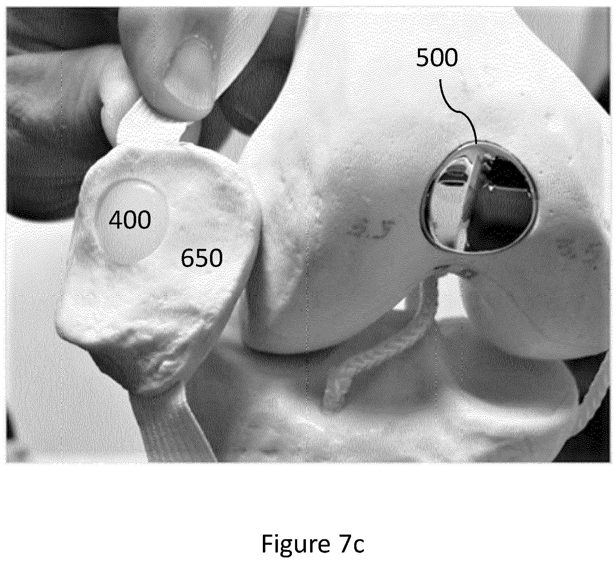

Figs. 7a-c illustrate a patellofemoral implant arrangement 700 for turning a patient's dysplastic or deformed patellofemoral joint into a healthier patellofemoral joint. The patellofemoral implant arrangement 700 preferably comprises a patellar implant 400, having an articulating surface 410, and a trochlear implant 500, having an articulating surface 510. The implants 400, 500 are illustrated as implanted into a knee joint, where the patella 650 lies in the intercondylar groove of the femur. Fig. 7c shows the patellofemoral implant arrangement in a 3D model of the knee joint.

It may be advantageous to double-check that the articulating surface 410 of the patellar implant 400 matches the articulating surface 510 of an opposing femoral trochlear implant 500, since the articulating surfaces of the patella and the trochlea would normally match each other in a healthy patellofemoral joint.

In one specific embodiment, the system 100 further comprises storage means 110 in which example curvatures of articulating surfaces in a healthy patellofemoral joint are stored.

In one specific embodiment, the system 100 the at least one processor 120 is arranged to retrieve example curvatures of articulating surfaces in a healthy patellofemoral joint from the storage means 110, and propose an improved curvature of the articulating surface of the patella and/or the femoral trochlea based on the retrieved curvatures.

In one specific embodiment, the system 100 for determining the shape and dimensions of at least one implant for turning a patient's dysplastic or deformed patellofemoral joint into a healthier patellofemoral joint, the system comprising at least one processor 120 arranged to: generate a first 3D model based on a series of radiological images of the patellofemoral joint; adapt the first 3D model into a second 3D model, by adapting a surface curvature of the first 3D model into a desired surface curvature; determine the shape and dimensions for at least one implant based on the second 3D model; and ensure that all the points around the circumference of said implant in the second 3D model correspond to points on the surface of the first 3D model.

In one specific embodiment, the the least one processor 120 of the system 100 is further arranged to determine the shape and dimensions for at least one guide tool based on the first 3D model.

In one specific embodiment, the at least one processor 120 of the system is further arranged to take into account also determined damage to the patellofemoral joint when determining the shape and dimensions for a patellar implant and/or a trochlear implant.

In one specific embodiment, the at least one processor 120 of the system is further arranged to output said determined shape and dimensions of the implant or implants as parameters for manufacturing said implant or implants.

### Method embodiments

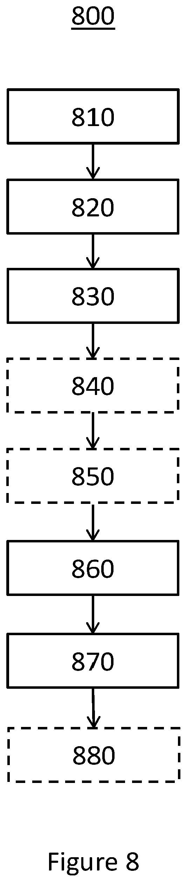

Fig. 8 is a flow diagram of embodiments of a method 800 for creating a healthy patellofemoral joint in a patient. In accordance with one or more embodiments, the method 800 comprises:
Step 810: analyzing the curvature of the patella in a patellofemoral joint.
Step 820: analyzing the curvature of the femoral trochlea in said patellofemoral joint.
Step 830: determining whether any of said analyzed curvatures indicate that the patellofemoral joint is dysplastic or deformed.
Step 860: if any of said analyzed curvatures indicate that the patellofemoral joint is dysplastic or deformed, proposing an improved curvature of the articulating surface of the patella and/or the femoral trochlea.
Step 870: determining the shape and dimensions for a patellar implant that would create said improved curvature of the articulating surface of the patella, and/or a trochlear implant that would create said improved curvature of the articulating surface of the femoral trochlea.

This enables the use of implants for improving dysplastic or deformed patellofemoral joints.

In embodiments, the determining 830 of whether any of said analyzed curvatures indicate that the patellofemoral joint is dysplastic or deformed involves determining whether the sulcus angle between the medial and lateral trochlear facets of the femur is larger than a threshold. The threshold may e.g. be 145-160 degrees, preferably 145-150 degrees. However, an experienced radiologist may be able to determine whether the patellofemoral joint is dysplastic or deformed without having to actually measure any angles (although such a measurement may form a part of this determination). A radiologist may e.g. when analyzing curvatures in a patellofemoral joint determine that an angle indicates a dysplastic or deformed patellofemoral joint, even without actually measuring any angles. An actual measurement of the sulcus angle typically involves placing the angle tip at the lowest point of the intercondylar groove of the femoral trochlea.

In embodiments, the sulcus angle is measured in radiological images (e.g. images provided by a magnetic resonance imaging (MRI) system, an x-ray imaging system, an ultrasonic imaging system, a fluoroscopic imaging system and/or a computer tomography (CT), e.g. a CBCT or Arthro-CT, system), or in a 3D model generated based on a series of radiological images.

In embodiments, if there is determined to be damage to the patella, the analyzing 810 of the curvature of the articulating surface of the patella comprises analyzing a simulated surface corresponding to the articulating surface that the patella would have had if it had not been damaged. This enables a determination of whether the patella is dysplastic or deformed, or simply damaged. If there is determined to be damage, an analysis of the damaged curvature may not provide correct results regarding whether the patella is dysplastic or deformed, and it is therefore preferred to instead analyze a simulated healthy surface.

In embodiments, if there is determined to be damage to the femoral trochlea, the analyzing 820 of the curvature of the articulating surface of the femoral trochlea comprises analyzing a simulated surface corresponding to the articulating surface that the femoral trochlea would have had if it had not been damaged. If there is determined to be damage, an analysis of the damaged curvature may not provide correct results regarding whether the femoral trochlea is dysplastic or deformed, and it is therefore preferred to instead analyze a simulated healthy surface. This enables a determination of whether the femoral trochlea is dysplastic or deformed, or simply damaged.

In embodiments, the determining 870 of the shape and dimensions for the at least one implant comprises taking into account also determined damage to the patellofemoral joint, e.g. by adapting parameters such as the implant area, the implant thickness, and/or the positioning of the implant.

In one or more embodiments, the method 800 further comprises one or more of the following steps:
Step 840: storing example curvatures of articulating surfaces in a patellofemoral joint in a storage means 110.
Step 850: retrieving example curvatures of articulating surfaces in a patellofemoral joint from the storage means 110. In this case, the proposing 860 of an improved curvature of the articulating surface of the patella and/or the femoral trochlea preferably involves creating the improved curvature based on the retrieved curvatures.
Step 880: outputting said determined shape and dimensions of the implant or implants as parameters for manufacturing said implant or implants. This enables the manufacturing of implants suitable for turning a patient's dysplastic or deformed patellofemoral joint into a healthier patellofemoral joint.

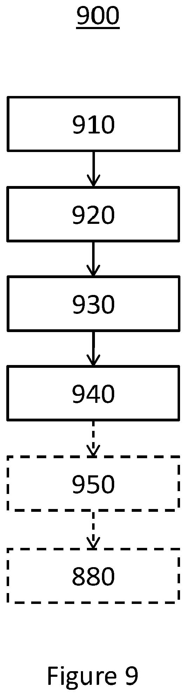

Fig. 9 is a flow diagram of embodiments of a method 900 for determining the shape and dimensions of at least one implant for turning a patient's dysplastic or deformed patellofemoral joint into a healthier patellofemoral joint. In accordance with one or more embodiments, the method 900 comprises:
Step 910: generating a first 3D model based on a series of radiological images of the patellofemoral joint.
Step 920: adapting the first 3D model into a second 3D model, by adapting a surface curvature of the first 3D model into a desired surface curvature.
Step 930: determining the shape and dimensions for at least one implant based on the second 3D model.
Step 940: ensuring that all the points around the circumference of said implant in the second 3D model correspond to points on the surface of the first 3D model.

In embodiments, the determining 930 of the shape and dimensions for the at least one implant comprises taking into account also determined damage to the patellofemoral joint, e.g. by adapting parameters such as the implant area, the implant thickness, and/or the positioning of the implant.

In one or more embodiments, the method 900 further comprises one or more of the following steps:
Step 880: outputting said determined shape and dimensions of the implant or implants as parameters for manufacturing said implant or implants. This enables the manufacturing of implants suitable for turning a patient's dysplastic or deformed patellofemoral joint into a healthier patellofemoral joint.
Step 950: determining the shape and dimensions for at least one guide tool based on the first 3D model.

In one specific embodiment, the method 900 for determining the shape and dimensions of at least one implant for turning a patient's dysplastic or deformed patellofemoral joint into a healthier patellofemoral joint, comprises: generating 910 a first 3D model based on a series of radiological images of the patellofemoral joint; adapting 920 the first 3D model into a second 3D model, by adapting a surface curvature of the first 3D model into a desired surface curvature; determining 930 the shape and dimensions for at least one implant based on the second 3D model; and ensuring 940 that all the points around the circumference of said implant in the second 3D model correspond to points on the surface of the first 3D model.

In one specific embodiment, the method further comprising determining 950 the shape and dimensions for at least one guide tool based on the first 3D model.

In one specific embodiment, the determining 870, 930 of the shape and dimensions for the at least one implant comprises taking into account also determined damage to the patellofemoral joint.

In one specific embodiment, the method further comprising outputting 880 said determined shape and dimensions of the implant or implants as parameters for manufacturing said implant or implants.

### Further embodiments

Where applicable, various embodiments provided by the present disclosure can be implemented using hardware, software, or combinations of hardware and software. Also where applicable, the various hardware components and/or software components set forth herein can be combined into composite components comprising software, hardware, and/or both without departing from the claimed scope of the present disclosure. Where applicable, the various hardware components and/or software components set forth herein can be separated into sub-components comprising software, hardware, or both without departing from the claimed scope of the present disclosure. In addition, where applicable, it is contemplated that software components can be implemented as hardware components, and vice-versa. The method steps of one or more embodiments described herein may be performed automatically, by any suitable processing unit, or one or more steps may be performed manually, with or without the use of a processing unit. Where applicable, the ordering of various steps described herein can be changed, combined into composite steps, and/or separated into sub-steps to provide features described herein.

Software in accordance with the present disclosure, such as program code and/or data, can be stored in non-transitory form on one or more machine-readable mediums. It is also contemplated that software identified herein can be implemented using one or more general purpose or specific purpose computers and/or computer systems, networked and/or otherwise.

In embodiments, there are provided a computer program product comprising computer readable code configured to, when executed in a processor, perform any or all of the method steps described herein. In some embodiments, there are provided a non-transitory computer readable memory on which is stored computer readable and computer executable code configured to, when executed in a processor, perform any or all of the method steps described herein.

In one or more embodiments, there is provided a non-transitory machine-readable medium on which is stored machine-readable code which, when executed by a processor, controls the processor to perform the method of any or all of the method embodiments presented herein.

The foregoing disclosure is not intended to limit the present invention to the precise forms or particular fields of use disclosed. It is contemplated that various alternate embodiments and/or modifications to the present invention, whether explicitly described or implied herein, are possible in light of the disclosure. Accordingly, the scope of the invention is defined only by the claims.

## Claims

1. System (100) for turning a patient's dysplastic or deformed patellofemoral joint into a healthier patellofemoral joint, the system comprising at least one processor (120) configured to:
receive or retrieve image data from medical images of the patella in a patellofemoral joint;
receive or retrieve image data from medical images of the femoral trochlea in said patellofemoral joint;
determine whether said image data indicates damage on the patellafermoral joint;
if damage on the patellafemoral joint is determined, determine if the patellofemoral joint is dysplastic or deformed, or just simply damaged;
if said image data indicates that the patellofemoral joint is dysplastic or deformed, determine an improved curvature of the articulating surface of the patella and/or the femoral trochlea based on a curvature of a simulated healthy surface of an articulating surface of the patella and a curvature of a simulated healthy surface of an articulating surface of the femoral trochlea; and
create one or more implants that will make the patellofemoral joint healthier by changing the curvature of at least one of the articulating surfaces by creating a patellar implant having a shape and dimensions configured to create said improved curvature of the articulating surface of the patella, and/or creating a trochlear implant having a shape and dimensions configured to create said improved curvature of the articulating surface of the femoral trochlea.

2. System (100) according to claim 1, wherein the determination of whether the patellofemoral joint is dysplastic or deformed involves determining whether the sulcus angle between the medial and lateral trochlear facets of the femur is larger than a threshold.

3. System (100) according to claim 2, wherein the threshold is 145-160 degrees, preferably 145-150 degrees.

4. System (100) according to claim 2 or 3, wherein the sulcus angle is measured in radiological images, or in a 3D model generated based on a series of radiological images.

5. System (100) according to any one of claims 1-4, wherein if there is determined to be damage to the patella, the curvature of the articulating surface of the patella that is analyzed is a simulated surface corresponding to the articulating surface that the patella would have had if it had not been damaged.

6. System (100) according to any one of claims 1-5, wherein if there is determined to be damage to the femoral trochlea, the curvature of the articulating surface of the femoral trochlea that is analyzed is a simulated surface corresponding to the articulating surface that the femoral trochlea would have had if it had not been damaged.

7. Method (800) for creating an implant
for turning a patient's dysplastic or deformed patellofemoral joint into a healthier patellofemoral joint, the method comprising:
receiving or retrieving image data from medical images of the patella in a patellofemoral joint;
receiving or retrieving image data from medical images of the femoral trochlea in said patellofemoral joint;
determining (830) whether said image data indicates damage on the patellafermoral joint;
if damage on the patellafermoral joint is determined, determining if the patellofemoral joint is dysplastic or deformed, or just simply damaged;
if said image data indicates that the patellofemoral joint is dysplastic or deformed, determining (860) an improved curvature of the articulating surface of the patella and/or the femoral trochlea based on a curvature of a simulated healthy surface of an articulating surface of the patella and a curvature of a simulated healthy surface of an articulating surface of the femoral trochlea; and
creating one or more implants that will make the patellofemoral joint healthier by changing the curvature of at least one of the articulating surfaces by creating (870) a patellar implant having a shape and dimensions configured to create said improved curvature of the articulating surface of the patella, and/or creating (870) a trochlear implant having a shape and dimensions configured to create said improved curvature of the articulating surface of the femoral trochlea

8. Method (800) according to claim 7, wherein the determining (830) of whether the patellofemoral joint is dysplastic or deformed involves determining whether the sulcus angle between the medial and lateral trochlear facets of the femur is larger than a threshold.

9. Method (800) according to claim 8, wherein the threshold is 145-160 degrees, preferably 145-150 degrees.

10. Method (800) according to claim 7 or 8, wherein the sulcus angle is measured in radiological images, or in a 3D model generated based on a series of radiological images.

11. Method (800) according to any one of claims 7 - 10, wherein if there is determined to be damage to the patella, the analyzing (810) of the curvature of the articulating surface of the patella comprises analyzing a simulated surface corresponding to the articulating surface that the patella would have had if it had not been damaged.

12. Method (800) according to any one of claims 7 - 11, wherein if there is determined to be damage to the femoral trochlea, the analyzing (820) of the curvature of the articulating surface of the femoral trochlea comprises analyzing a simulated surface corresponding to the articulating surface that the femoral trochlea would have had if it had not been damaged.

13. Method (800) according to any one of claims 7 - 12, further comprising storing (840) example curvatures of articulating surfaces in a healthy patellofemoral joint in a storage means (110).

14. Method (800) according to claim 13, further comprising retrieving (850) example curvatures of articulating surfaces in a healthy patellofemoral joint from the storage means (110), wherein the proposing (860) of an improved curvature of the articulating surface of the patella and/or the femoral trochlea involves creating the improved curvature based on the retrieved curvatures.

15. Non-transitory machine-readable medium on which is stored machine-readable code which, when executed by at least one processor (120), controls the processor to perform the method of any one of claims 7 - 14.

## Patentansprüche

1. System (100) zum Umwandeln des dysplastischen oder deformierten Patellofemoralgelenks eines Patienten in ein gesünderes Patellofemoralgelenk, wobei das System mindestens einen Prozessor (120) umfasst, der zum Folgenden ausgebildet ist:
Bilddaten aus medizinischen Bildern der Patella in einem Patellofemoralgelenk zu empfangen oder abzurufen;
Bilddaten aus medizinischen Bildern der femoralen Trochlea im Patellofemoralgelenk zu empfangen oder abzurufen;
zu bestimmen, ob die Bilddaten eine Schädigung am Patellofemoralgelenk anzeigen;
wenn eine Schädigung am Patellofemoralgelenk bestimmt wird, zu bestimmen, ob das Patellofemoralgelenk dysplastisch oder deformiert oder nur einfach geschädigt ist;
wenn die Bilddaten anzeigen, dass das Patellofemoralgelenk dysplastisch oder deformiert ist, eine verbesserte Krümmung der Gelenkfläche der Patella und/oder der femoralen Trochlea basierend auf einer Krümmung einer simulierten gesunden Fläche einer Gelenkfläche der Patella und einer Krümmung einer simulierten gesunden Fläche einer Gelenkfläche der femoralen Trochlea zu bestimmen; und
ein oder mehrere Implantate zu erzeugen, die das Patellofemoralgelenk gesünder machen werden, indem die Krümmung von mindestens einer der Gelenkflächen durch Erzeugen eines Patellaimplantats, das eine Form und Abmessungen aufweist, die ausgebildet sind, die verbesserte Krümmung der Gelenkfläche der Patella zu erzeugen, und/oder durch Erzeugen eines Trochleaimplantats, das eine Form und Abmessungen aufweist, die ausgebildet sind, die verbesserte Krümmung der Gelenkfläche der femoralen Trochlea zu erzeugen, verändert wird.

2. System (100) nach Anspruch 1, wobei die Bestimmung, ob das Patellofemoralgelenk dysplastisch oder deformiert ist, das Bestimmen enthält, ob der Sulkuswinkel zwischen den medialen und lateralen Trochleafacetten des Femurs größer als ein Schwellenwert ist.

3. System (100) nach Anspruch 2, wobei der Schwellenwert 145-160 Grad, bevorzugt 145-150 Grad, ist.

4. System (100) nach Anspruch 2 oder 3, wobei der Sulkuswinkel in radiologischen Bildern oder in einem 3D-Modell gemessen wird, das basierend auf einer Reihe radiologischer Bilder erzeugt wird.

5. System (100) nach einem der Ansprüche 1-4, wobei, wenn bestimmt wird, dass eine Schädigung der Patella vorliegt, die Krümmung der Gelenkfläche der Patella, die analysiert wird, eine simulierte Fläche ist, die der Gelenkfläche entspricht, die die Patella aufgewiesen hätte, wenn sie nicht geschädigt worden wäre.

6. System (100) nach einem der Ansprüche 1-5, wobei, wenn bestimmt wird, dass eine Schädigung der femoralen Trochlea vorliegt, die Krümmung der Gelenkfläche der femoralen Trochlea, die analysiert wird, eine simulierte Fläche ist, die der Gelenkfläche entspricht, die die femorale Trochlea aufgewiesen hätte, wenn sie nicht geschädigt worden wäre.

7. Verfahren (800) zum Erzeugen eines Implantats zum Umwandeln des dysplastischen oder deformierten Patellofemoralgelenks eines Patienten in ein gesünderes Patellofemoralgelenk, wobei das Verfahren Folgendes umfasst:
Empfangen oder Abrufen von Bilddaten aus medizinischen Bildern der Patella in einem Patellofemoralgelenk;
Empfangen oder Abrufen von Bilddaten aus medizinischen Bildern der femoralen Trochlea im Patellofemoralgelenk;
Bestimmen (830), ob die Bilddaten eine Schädigung am Patellofemoralgelenk anzeigen;
wenn eine Schädigung am Patellofemoralgelenk bestimmt wird, Bestimmen, ob das Patellofemoralgelenk dysplastisch oder deformiert oder nur einfach geschädigt ist;
wenn die Bilddaten anzeigen, dass das Patellofemoralgelenk dysplastisch oder deformiert ist, Bestimmen (860) einer
verbesserten Krümmung der Gelenkfläche der Patella und/oder der femoralen Trochlea basierend auf einer Krümmung einer simulierten gesunden Fläche einer Gelenkfläche der Patella und einer Krümmung einer simulierten gesunden Fläche einer Gelenkfläche der femoralen Trochlea; und Erzeugen eines oder mehrerer Implantate, die das Patellofemoralgelenk gesünder machen werden, indem die Krümmung von mindestens einer der Gelenkflächen durch Erzeugen (870) eines Patellaimplantats, das eine Form und Abmessungen aufweist, die ausgebildet sind, die verbesserte Krümmung der Gelenkfläche der Patella zu erzeugen, und/oder durch Erzeugen (870) eines Trochleaimplantats, das eine Form und Abmessungen aufweist, die ausgebildet sind, die verbesserte Krümmung der Gelenkfläche der femoralen Trochlea zu erzeugen, verändert wird.

8. Verfahren (800) nach Anspruch 7, wobei das Bestimmen (830), ob das Patellofemoralgelenk dysplastisch oder deformiert ist, das Bestimmen umfasst, ob der Sulkuswinkel zwischen den medialen und lateralen Trochleafacetten des Femurs größer als ein Schwellenwert ist.

9. Verfahren (800) nach Anspruch 8, wobei der Schwellenwert 145-160 Grad, bevorzugt 145-150 Grad, ist.

10. Verfahren (800) nach Anspruch 7 oder 8, wobei der Sulkuswinkel in radiologischen Bildern oder in einem 3D-Modell gemessen wird, das basierend auf einer Reihe radiologischer Bilder erzeugt wird.

11. Verfahren (800) nach einem der Ansprüche 7 - 10, wobei, wenn bestimmt wird, dass eine Schädigung der Patella vorliegt, das Analysieren (810) der Krümmung der Gelenkfläche der Patella das Analysieren einer simulierten Fläche umfasst, die der Gelenkfläche entspricht, die die Patella aufgewiesen hätte, wenn sie nicht geschädigt worden wäre.

12. Verfahren (800) nach einem der Ansprüche 7 - 11, wobei, wenn bestimmt wird, dass eine Schädigung der femoralen Trochlea vorliegt, das Analysieren (820) der Krümmung der Gelenkfläche der femoralen Trochlea das Analysieren einer simulierten Fläche umfasst, die der Gelenkfläche entspricht, die die femorale Trochlea aufgewiesen hätte, wenn sie nicht geschädigt worden wäre.

13. Verfahren (800) nach einem der Ansprüche 7 - 12, weiter umfassend Speichern (840) von beispielhaften Krümmungen von Gelenkflächen in einem gesunden Patellofemoralgelenk in einem Speichermittel (110).

14. Verfahren (800) nach Anspruch 13, weiter umfassend Abrufen (850) von beispielhaften Krümmungen von Gelenkflächen in einem gesunden Patellofemoralgelenk aus dem Speichermittel (110), wobei das Vorschlagen (860) einer verbesserten Krümmung der Gelenkfläche der Patella und/oder der femoralen Trochlea das Erzeugen der verbesserten Krümmung, basierend auf den abgerufenen Krümmungen, umfasst.

15. Nichttransitorisches maschinenlesbares Medium, auf dem maschinenlesbarer Code gespeichert ist, der, wenn er durch mindestens einen Prozessor (120) ausgeführt wird, den Prozessor steuert, das Verfahren nach einem der Ansprüche 7 - 14 auszuführen.

## Revendications

1. Système (100) destiné à transformer l'articulation fémoro-patellaire dysplasique ou déformée d'un patient en une articulation fémoro-patellaire plus saine, le système comprenant au moins un processeur (120) agencé pour :
recevoir ou récupérer des données d'image à partir d'images médicales de la patella dans une articulation fémoro-patellaire ;
recevoir ou récupérer des données d'image à partir d'images médicales de la trochlée fémorale dans ladite articulation fémoro-patellaire ;
déterminer si lesdites données d'image indiquent des dommages au niveau de l'articulation fémoro-patellaire ;
si des dommages sur l'articulation fémoro-patellaire sont déterminés, déterminer si l'articulation fémoro-patellaire est dysplasique ou déformée, ou simplement endommagée ;
si lesdites données d'image indiquent que l'articulation fémoro-patellaire est dysplasique ou déformée, déterminer une courbure améliorée de la surface articulaire de la patella et/ou de la trochlée fémorale sur la base d'une courbure d'une surface saine simulée d'une surface articulaire de la patella et d'une courbure d'une surface saine simulée d'une surface articulaire de la trochlée fémorale ; et
créer un ou plusieurs implants qui rendront l'articulation fémoro-patellaire plus saine en modifiant la courbure d'au moins une des surfaces articulaires en créant un implant patellaire présentant une forme et des dimensions agencées pour créer ladite courbure améliorée de la surface articulaire de la patella, et/ou en créant un implant trochléen présentant une forme et des dimensions agencées pour créer ladite courbure améliorée de la surface articulaire de la trochlée fémorale.

2. Système (100) selon la revendication 1, dans lequel la détermination de savoir si l'articulation fémoro-patellaire est dysplasique ou déformée implique de déterminer si l'angle de sillon entre les facettes trochléennes médiale et latérale du fémur est supérieur à un seuil.

3. Système (100) selon la revendication 2, dans lequel le seuil est de 145 à 160 degrés, de préférence 145 à 150 degrés.

4. Système (100) selon la revendication 2 ou la revendication 3, dans lequel l'angle de sillon est mesuré dans des images radiologiques, ou dans un modèle 3D généré sur la base d'une série d'images radiologiques.

5. Système (100) selon l'une quelconque des revendications 1 à 4, dans lequel, s'il est déterminé qu'il y a des dommages à la patella, la courbure de la surface articulaire de la patella qui est analysée est une surface simulée correspondant à la surface articulaire que la patella aurait présentée si elle n'avait pas été endommagée.

6. Système (100) selon l'une quelconque des revendications 1 à 5, dans lequel, s'il est déterminé qu'il y a des dommages à la trochlée fémorale, la courbure de la surface articulaire de la trochlée fémorale qui est analysée est une surface simulée correspondant à la surface articulaire que la trochlée fémorale aurait présentée si elle n'avait pas été endommagée.

7. Procédé (800) destiné à créer un implant pour transformer l'articulation fémoro-patellaire dysplasique ou déformée d'un patient en une articulation fémoro-patellaire plus saine, le procédé comprenant :
recevoir ou récupérer des données d'image à partir d'images médicales de la patella dans une articulation fémoro-patellaire ;
recevoir ou récupérer des données d'image à partir d'images médicales de la trochlée fémorale dans ladite articulation fémoro-patellaire ;
déterminer (830) si lesdites données d'image indiquent des dommages au niveau de l'articulation fémoro-patellaire ;
si des dommages au niveau de l'articulation fémoro-patellaire sont déterminés, déterminer si l'articulation fémoro-patellaire est dysplasique ou déformée, ou simplement endommagée ;
si lesdites données d'image indiquent que l'articulation fémoro-patellaire est dysplasique ou déformée, déterminer (860) une courbure améliorée de la surface articulaire de la patella et/ou de la trochlée fémorale sur la base d'une courbure d'une surface saine simulée d'une surface articulaire de la patella et d'une courbure d'une surface saine simulée d'une surface articulaire de la trochlée fémorale ; et
créer un ou plusieurs implants qui rendront l'articulation fémoro-patellaire plus saine en modifiant la courbure d'au moins une des surfaces articulaires en créant (870) un implant patellaire présentant une forme et des dimensions agencées pour créer ladite courbure améliorée de la surface articulaire de la patella, et/ou en créant (870) un implant trochléen présentant une forme et des dimensions agencées pour créer ladite courbure améliorée de la surface articulaire de la trochlée fémorale.

8. Procédé (800) selon la revendication 7, dans lequel la détermination (830) de savoir si l'articulation fémoro-patellaire est dysplasique ou déformée implique de déterminer si l'angle de sillon entre les facettes trochléennes médiale et latérale du fémur est supérieur à un seuil.

9. Procédé (800) selon la revendication 8, dans lequel le seuil est de 145 à 160 degrés, de préférence 145 à 150 degrés.

10. Procédé (800) selon la revendication 7 ou la revendication 8, dans lequel l'angle de sillon est mesuré dans des images radiologiques, ou dans un modèle 3D généré sur la base d'une série d'images radiologiques.

11. Procédé (800) selon l'une quelconque des revendications 7 à 10, dans lequel, s'il est déterminé qu'il y a des dommages à la patella, l'analyse (810) de la courbure de la surface articulaire de la patella comprend l'analyse d'une surface simulée correspondant à la surface articulaire que la patella aurait présentée si elle n'avait pas été endommagée.

12. Procédé (800) selon l'une quelconque des revendications 7 à 11, dans lequel, s'il est déterminé qu'il y a des dommages à la trochlée fémorale, l'analyse (820) de la courbure de la surface articulaire de la trochlée fémorale comprend l'analyse d'une surface simulée correspondant à la surface articulaire que la trochlée fémorale aurait présentée si elle n'avait pas été endommagée.

13. Procédé (800) selon l'une quelconque des revendications 7 à 12, comprenant en outre le stockage (840) de courbures d'exemple de surfaces articulaires dans une articulation fémoro-patellaire saine dans un moyen de stockage (110).

14. Procédé (800) selon la revendication 13, comprenant en outre la récupération (850) de courbures d'exemple de surfaces articulaires dans une articulation fémoro-patellaire saine à partir du moyen de stockage (110), dans lequel la proposition (860) d'une courbure améliorée de la surface articulaire de la patella et/ou de la trochlée fémorale implique de créer la courbure améliorée sur la base des courbures récupérées.

15. Support lisible par machine non transitoire sur lequel est stocké un code lisible par machine qui, lorsqu'il est exécuté par au moins un processeur (120), commande au processeur de réaliser le procédé de l'une quelconque des revendications 7 à 14.
